# EUROPÄISCHE PATENTANMELDUNG

(11) **EP 1 437 052 A1**
(43) Veröffentlichungstag der Anmeldung: **14.07.2004**
(21) Anmeldenummer: 04100041.5
(22) Anmeldetag: 08.01.2004
(51) Int. Cl.: A23L 1/304

(54) **Ocuvite Zink**

(30) Priorität: 09.01.2003 DE 20300304 U
(71) Anmelder: Dr. GERHARD MANN chem.-pharm. Fabrik GmbH, D-13581 Berlin (DE)
(72) Erfinder: Bellmann, Günther Dr., 13593 Berlin (DE); Claus-Herz, Gudrun Dr., 14167 Berlin (DE)
(74) Vertreter: Kietzmann, Lutz

(57) **Zusammenfassung**

Mikronährstoffkombinationsprodukt, wobei das Mikronährstoffkombinationsprodukt Zink und Kupfer in Form von Zinkgluconat und Kupfer(II)-gluconat umfasst. Das Mikronährstoffkombinationsprodukt kann beispielsweise für die nutritive Ergänzung bei altersbedingter Makuladegeneration verwendet werden.

## Beschreibung

Die vorliegende Erfindung betrifft ein Mikronährstoffkombinationsprodukt. Das Mikronährstoffkombinationsprodukt kann beispielsweise für die nutritive Ergänzung bei altersbedingter Makuladegeneration verwendet werden.

Es ist allgemein bekannt, dass das Auge ein Hochleistungsorgan ist, das durch den Sehprozess ständigen hohen Belastungen ausgesetzt ist. Durch Licht- und Sauerstoffeinwirkung entstehen sogenannte "freie Radikale", die zu krankhaften Veränderungen in der Netzhaut führen können.

Weiterhin ist bekannt, dass viele äußere Einflüsse, z. B. eine unausgewogene Ernährung, Rauchen und Alkoholgenuss, Umweltverschmutzung, Stress oder UV-Strahlung, zu einer Freisetzung von Radikalen führen können. Aber auch die normalen Vorgänge im Stoffwechsel setzen kurzfristig hochaktive Substanzen frei, die den Körper belasten und schädigen können. Zum Abbau dieser "freien Radikale" besitzt der Körper ein eigenes "antioxidatives" Schutzsystem, an dem Zink entscheidend beteiligt ist. Zudem ist Zink Bestandteil von mehr als 300 Enzymsystemen des Stoffwechsels, insbesondere ist die Retinol-Dehydrogenase Zink-abhängig.

Das Auge ist durch den Sehprozess und die ständige Lichteinwirkung in erhöhtem Maß oxidativem Stress ausgesetzt. Das Auge, und insbesondere die Netzhaut und die Makula, haben einen erhöhten Bedarf an einer ausreichenden Versorgung mit antioxidativen Substanzen und Zink. Unter dem medizinischen Begriff "Makula lutea" ist die Netzhautmitte, also der Punkt des schärfsten Sehens zu verstehen. Hier befinden sich die empfindlichsten Sehzellen des Auges. Bei einer länger anhaltenden Mangelversorgung der Makula mit Mikronährstoffen steigt das Risiko der Entwicklung einer "altersbedingten Makuladegeneration" (AMD).

Insbesondere im Alter kann sich ein Mangel an Zink durch Unterversorgung aufgrund von Mangel- oder Fehlernährung oder Krankheit rasch bemerkbar machen. Die Versorgung der Makula des Auges, die eine zentrale Funktion beim Sehprozess einnimmt, ist von großer Bedeutung. Der Vorbeugung einer AMD wird durch darauf abgestimmte Präparate zu wenig Rechnung getragen.

Im Stand der Technik bekannte Zinkpräparate zur Nahrungsergänzung sind häufig hochdosierte Präparate, die hohe Mengen an Zinksulfat enthalten. Diese Präparate haben den Nachteil, dass sie für eine Dauertherapie beziehungsweise für eine zeitlich unbegrenzte Prophylaxe nicht geeignet sind, da die Dosis dieser Präparate zu Überdosierungen und gesundheitsschädlichen Nebenwirkungen führen können. Eine akute Überdosierung an Zinksulfat kann zu Übelkeit, Brechreiz oder Magenkrämpfen führen. Überschreitet die Überdosierung die therapeutische Dosierung um ein Vielfaches, kann es zu Symptomen wie Kopfschmerzen, Diarrhöe oder Erbrechen führen. Eine langfristige höher dosierte (> 25 mg Zink/Tag) Zinkeinnahme kann zu Kupfermangel führen, der durch anämische Zustände gekennzeichnet ist. Weiterhin ist auch die Verwendung von Zink und Kupfer in Form ihrer Hefen umstritten.

Eine Reduktion der alimentären Zinkzufuhr kann andererseits rasch zu einem Zinkmangel führen, da es keine Organe mit ausgeprägter Speicherfähigkeit für Zink im menschlichen Organismus gibt.

Es besteht daher Bedarf an einem Mittel, das speziell als Nahrungsergänzungsmittel zur Stärkung des Auges entwickelt wurde und insbesondere einer Mangelversorgung der Makula mit Zink entgegenwirkt. Darüber hinaus besteht Bedarf an einem Mittel, das als solches keine gesundheitsschädliche Nebenwirkung hervorruft und sowohl unbedenklich zur Präventionen von Mangelerscheinungen dienen kann, wie auch bei bereits bestehender altersbedingter Makuladegeneration verwendbar ist.

Es ist daher die Aufgabe der vorliegenden Erfindung, ein Mittel zur Verfügung zu stellen, das die vorgenannten Nachteile des Standes der Technik überwindet. Insbesondere ist es die Aufgabe der vorliegenden Erfindung, ein Mittel zur Verfügung zu stellen, das eine umfassende Versorgung der Makula mit Zink bietet und gleichzeitig durch die Vermeidung einer Überdosierung Nebenwirkungen vermeidet.

Diese Aufgabe wird durch einMikronährstoffkombinationsprodukt gelöst, wobei das Mikronährstoffkombinationsprodukt Zink und Kupfer in Form von Zinkgluconat und Kupfer(II)-gluconat umfasst.

Es hat sich überraschend gezeigt, dass durch Verwendung von Zink- und Kupfer(II)-gluconat die Bioverfügbarkeit des Zinks gegenüber der Verwendung von Zinksulfat verbessert war. Es wird vermutet, dass die Versorgung der Makula mit Zink durch Verwendung von Zinkgluconat verbessert wird. Es ist weiterhin denkbar, dass durch die Verwendung des gut resorbierbaren Zinkgluconats die notwendige Zinkmenge des Mikronährstoffkombinationsproduktes reduziert werden kann, und damit eine mögliche Überdosierung vermieden werden kann.

Das erfindungsgemäß verwendete Zinkgluconat zeigt eine bessere Resorption als das häufig verwendete Zinksulfat. Diese erhöhte Bioverfügbarkeit ermöglicht, dass eine weitaus geringere Menge an Zink als Tagesdosis bzw. Einzeldosis eingesetzt werden kann, wodurch die Gefahr von Nebenwirkungen deutlich gesenkt wird. Zinkgluconat zeigt insbesondere eine deutlich bessere Resorption als das auch häufig verwendete Zinkoxid.

Weiterhin kann durch eine Verwendung von Zinkgluconat auf eine Zugabe größerer Mengen an Hilfsstoffen verzichtet werden. Dies ergibt einen großen Vorteil der erfindungsgemäßen Formulierung, die eine Verkleinerung der Darreichungsformen, insbesondere der Tabletten, und somit eine leichtere Schluckbarkeit ermöglicht.

Vorteilhafter Weise umfasst das hinsichtlich seiner Bestandteile optimiert formulierte Mikronährstoffkombinationsprodukt, bezogen auf 100 g des Mikronährstoffkombinationsproduktes, die Wirkstoffe:
a. 1-10 g, bevorzugt 5-9 g, Zink;
b. 100-300 mg, bevorzugt 150-200 mg, Kupfer.

Erfindungsgemäß wurde gefunden, dass sich Zink sehr gut für die diätetische Prophylaxe einer Makuladegeneration eignet. Die gezielte Zufuhr einer ausreichenden Konzentration dieses Spurenelementes kann folglich dazu beitragen, einem Zinkmangel entgegenzuwirken. Es ist denkbar, das Auge somit vor der Entstehung einer altersbedingten Makuladegeneration zu schützen oder deren Fortschreiten zu verlangsamen.

Das erfindungsgemäße Mikronährstoffkombinationsprodukt ist kein Arzneimittel. Es kann zur unterstützenden nutritiven Ergänzung oder zur begleitenden Verabreichung beispielsweise bei der Behandlung einer altersbedingten Makuladegeneration verwendet werden.

Vorteilhaft ist darüber hinaus, dass die Dosierungen des erfindungsg emäßen Mittels keine schädigenden Nebenwirkungen verursachen und zur Prophylaxe wie auch zum Ausgleich einer bestehenden Mangelversorgung der Makula dienen können. Dies ist zur nutritven wie auch zur diätetischen Behandlung bei altersbedingter Makuladegeneration vorteilhaft.

Bevorzugt weist das erfindungsgemäße Mikronährstoffkombinationsprodukt keine antioxidativen Vitamine auf.

Das erfindungsgemäße Mikronährstoffkombinationsprodukt eignet sich insbesondere zur additiven diätetischen Ergänzung von Mikronährstoffkombinationsprodukten mit antioxidativ wirkenden Inhaltsstoffen. Eine zeitversetzte Gabe verbessert die Bioverfügbarkeit von Zink, welches Bestandteil antioxidativ wirkender Enzyme ist, und es ist denkbar, dass sich somit eine synergistische Wirkung entfalten kann.

Das erfindungsgemäße Mikronährstoffkombinationsprodukt kann durch das Spurenelement Zink die antioxidativen Eigenschaften geeigneter Präparate unterstützen und in Verbindung mit geeigneten Mikronährstoffkombinationsprodukten eine deutliche Verbesserung der Eigenschaften z. B. von Vitaminen und Carotinoiden bewirken. Der Entstehung einer Mangelsituation an Zink kann vorgebeugt werden.

Weiterhin weist das erfindungsgemäße Mikronährstoffkombinationsprodukt Kupfer in Form von Kupfer(II)-gluconat auf, welches einem möglichen Kupfermangel, der aus der Einnahme von Zink resultieren kann, entgegenwirkt. Vorteilhaft ist, dass das erfindungsgemäße Mikronährstoffkombinationsprodukt aufgrund des erfindungsgemäß ebenfalls niedrigen Zinkanteils und der resultierenden deutlichen Verringerung der durch Zink verursachten Nebenwirkungen, einen äußerst gering dosierten Anteil Kupfer umfasst.

Das Mikronährstoffkombinationsprodukt kann fest-, flüssig und/oder gelförmig vorliegen, vorzugsweise liegt das Mikronährstoffkombinationsprodukt in Präparatformen, ausgewählt aus der Gruppe umfassend Tabletten, Kapseln, Pulver, Granulat, Lösungen und/oder Brausetabletten mit gleicher oder unterschiedlicher Zusammensetzung vor. Besonders bevorzugt liegt das Mikronährstoffkombinationsprodukt in Form von Tabletten vor, wobei die Tablette gewölbt und mit einer Bruchkerbe ausgeführt werden kann. Diese Bruchkerbe ermöglicht in vorteilhafter Weise eine Verteilung der erfindungsgemäßen Dosierung auf zwei zeitlich getrennte Zeitpunkte der Einnahme.

Ganz besonders bevorzugt liegt das Mikronährstoffkombinationsprodukt in Form kleiner, leicht schluckbarer Tabletten vor, welche einen Durchmesser von weniger als 10 mm, noch bevorzugter 7 mm, ein Gewicht von weniger als 200 mg, noch bevorzugter 140 mg, eine Härte von 20-100 N, noch bevorzugter 40-60 N und/oder eine Dicke von weniger als 5 mm, noch bevorzugter weniger als 3,2 mm, aufweisen. Der Feuchtigkeitsgehalt der pressfertigen Masse beträgt 20-40 %, bevorzugt 30,0 % ± 7,5 % (RF) relative Feuchte. Weiterhin ist die pressfertige Masse vorteilhafter Weise sehr gut mit Rundläufertabletten-Pressen verpressbar.

Das Mikronährstoffkombinationsprodukt kann Zusätze aufweisen, die die Bioverfügbarkeit, Löslichkeit und/oder Lösegeschwindigkeit verbessern. Ferner kann das Mikronährstoffkombinationsprodukt Füllstoffe, Formentrennmittel, Zerfallsbeschleuniger, Sprengmittel, Stoffe, die die Haltbarkeit verbessern, geschmacksmaskierende Stoffe, geschmacksverbessernde Stoffe und/oder Überzugsmittel aufweisen. Bevorzugt weist das erfindungsgemäße Mikronährstoffkombinationsprodukt Füllstoffe, Formentrennmittel und/oder Zerfallsbeschleuniger auf.

Geeignet sind übliche, dem Fachmann bekannte Zusätze, bevorzugte Füllstoffe sind Cellulose und/oder Lactose, bevorzugte Formentrennmittel sind Metallseifen, besonders bevorzugt ist Magnesiumstearat. Als Zerfallsbeschleuniger finden bevorzugt Stärkederivate, Cellulosederivate und/oder quervernetztes Polyvinylpyrrolidon Anwendung, besonders bevorzugt ist Polyplas done XL.

Zur Maskierung oder Verbesserung des Geschmacks können Stoffe ausgewählt aus der Gruppe umfassend natürliche, naturidentische oder synthetische Aromastoffe, Essenzen, ätherische Öle, natürliche oder synthetische Glutamate, Inosate, Guanylate und/oder Glycin oder deren Mischungen eingesetzt werden. Bevorzugt zur Verbesserung des Geschmacks sind Fruchtaromen. Weitere bevorzugte Zusätze sind Farbstoffe, Konservierungsmittel, Säuerungsmittel, Emulgatoren, Stabilisatoren, Süßungsmittel und/oder Festig ungsmittel.

Die erfindungsgemäßen Darreichungsformen können weiterhin in der Galenik übliche, pharmakologisch unbedenkliche und mit Gluconat verträgliche Hilfsstoffe aufweisen.

In einer besonders bevorzugten Ausführungsform weist das Mikronährstoffkombinationsprodukt Zinkgluconat, Kupfer(II)-gluconat, Cellulose, Lactose, Polyvinylpolypyrrolidon und/oder Magnesiumstearat auf.

In einer bevorzugten Ausführungsform umfasst das Mikronährstoffkombinationsprodukt, bezogen auf 100 g des Mikronährstoffkombinationsproduktes, die Wirkstoffe:
a. 7,1 g Zink;
b. 179 mg Kupfer.

In einer weiterhin bevorzugten Ausführungsform weist das Mikronährstoffkombinationsprodukt, bezogen auf 100 g des Mikronährstoffkombinationsproduktes, weniger als 1 g Eiweiß, weniger als 1 g Fett und/oder 14,3 g Kohlenhydrate auf. Weiterhin bevorzugt weist das Mikronährstoffkombinationsprodukt, bezogen auf 100 g des Mikronährstoffkombinationsproduktes, 1,2 Broteinheiten und/oder 204 kcal oder 875 kJ auf.

In einer weiteren bevorzugten Ausführu ngsform umfasst das Mikronährstoffkombinationsprodukt, bezogen auf eine Einzeldosis, die Wirkstoffe:
a. 10 mg Zink;
b. 250 µg Kupfer.

Unter einer Einzeldosis wird im Sinne dieser Anmeldung eine Verabreichungseinheit des Mikronährstoffkombinationsproduktes verstanden, eine Einzeldosis des Mikronährstoffkombinationsproduktes entspricht beispielsweise einer Tablette, einer Kapsel oder einer einzelnen Verabreichungseinheit einer anderen Präparatform, wie Pulver oder Granulat. Unter einer Tagesdosis wird im Sinne dieser Anmeldung die Menge des Mikronährstoffkombinationsproduktes verstanden, die pro Tag verabreicht wird.

In einer bevorzugten Ausführungsform weist das Mikronährstoffkombinationsprodukt, bezogen auf eine Einzeldosis, weniger als 0,01 g Eiweiß, weniger als 0,01 g Fett und/oder 0,02 g Kohlenhydrate auf. Weiterhin bevorzugt weist das Mikronährstoffkombinationsprodukt, bezogen auf eine Einzeldosis, 0,002 Broteinheiten und/oder 0,3 kcal oder 1,2 kJ auf.

Das Mikronährstoffpräparat kann auch Zusätze, Begleitstoffe und/oder Rohstoffe enthalten, die schwerer sind als die eigentlichen Wirkstoffe, so dass die angegebenen Mengen bezogen auf eine Tagesdosis bzw. Einzeldosis niedriger oder auch höher sein können.

Die Tagesdosis und/oder Einzeldosis ist vorzugsweise auf mehrere gleiche oder unterschiedliche Präparatformen verteilt, wobei die Präparatformen gleiche oder unterschiedliche Wirkstoffe und/oder Wirkstoffgewichtsgehalte aufweisen können.

Das Mikronährstoffkombinationsprodukt kann zur Herstellung eines Mittels zur diätetischen Verhütung und Behandlung von Augenkrankheiten, bevorzugt bei altersbedingter Makuladegeneration verwendet werden. Das erfindungsgemäße Mikronährstoffkombinationsprodukt ist weiterhin geeignet als Mittel zur Nahrungsergänzung.

Der Begriff "diätetische Behandlung" ist in der EU-Richtlinie 1999/21/EG, die seit 01.01.2002 als 10. Verordnung zur Änderung der Diätverordnung in deutsches Recht umgesetzt worden ist, näher erläutert.

Durch die vorteilhaften Wirkstoffe des Mikronährstoffkombinationsprodukts kann dieses auch im Rahmen einer diätetischen Emährungsberatung als Lebensmittel, insbesondere Nahrungsergänzungsmittel eingesetzt werden. Bevorzugt kann das Mikronährstoffkombinationsprodukt als nutritive Ergänzung zur begleitenden bzw. unterstützenden Verabreichung eingesetzt werden. Es hat sich gezeigt, dass insbesondere ein drohender Zinkmangel bei älteren Menschen ausgeglichen werden kann.

In seiner Funktion als Nahrungsergänzungsmittel weist das erfindungsgemäße Mikronährstoffkombinationsprodukt das Spurenelement Zink auf, das durch eine ausgeglichene Versorgung der Makula zur Verbesserung der Sehfähigkeit im Alter positiv beitragen kann.

### Beispiele für Mikronährstoffkombinationsprodukte sind nachstehend angegeben:

Es versteht sich, dass die Präparatformen, ausgewählt aus der Gruppe umfassend Tabletten, Kapseln, Pulver, Granulat, Lösungen und/oder Brausetabletten, die üblichen zur Formulierung der jeweiligen Präparatformen eingesetzten Hilfsstoffe aufweisen, so dass in den Beispielen lediglich die enthaltenen Wirkstoffe aufgeführt sein können.

### Beispiel 1

Mikronährstoffkombinationsprodukt umfassend 1 Tablette mit folgenden Wirkstoffen:
- 10 mg Zink;
- 250 µg Kupfer.

### Beispiel 2

Rezeptur für 1 Tablette ä 140 mg, umfassend:
- 73,17 mg Zinkgluconat, Granulat;
- 1,87 mg Kupfer(II)-gluconat, Pulver;
- 40,04 mg Cellulose, mikrokristallin;
- 20,02 mg Lactose;
- 3,5 mg Polyplasdone XL;
- 1,4 mg Magnesiumstearat,
wobei die Angaben für Zinkgluconat und Kupfer(II)-gluconat einen Zuschlag von 5 % aufweisen. Die Angaben beziehen sich auf die getrocknete Substanz.

### Beispiel 3

Rezeptur für 210 kg oder 1.500.000 Tabletten, umfassend pro 1.500.000 Tabletten:
- 109,755 kg Zinkgluconat, Granulat;
- 2,805 kg Kupfer(II)-gluconat, Pulver;
- 60,060 kg Cellulose, mikrokristallin;
- 30,030 kg Lactose;
- 5,250 kg Polyplasdone XL;
- 2,1 kg Magnesiumstearat.

Der Feuchtigkeitsgehalt der pressfertigen Masse wurde mit einem rotronic Hygroskop ermittelt und betrug 30,0 % ± 7,5 % (RF) relative Feuchte. Die pressfertige Masse wurde folgend auf einer Korsch PH 343 zu Tabletten verpresst, welche folgende Eigenschaften aufwiesen:
- Durchmesser: 7 mm, gewölbt mit Bruchkerbe;
- Gewicht: 140 mg;
- Härte: 40-60 N;
- Dicke: < 3,2 mm.

Die Einstellung der Maschinen erfolgte nach SOP P-001.

## Patentansprüche

1. Mikronährstoffkombinationsprodukt, **dadurch gekennzeichnet dass** das Mikronährstoffkombinationsprodukt Zink und Kupfer in Form von Zinkgluconat und Kupfer(II)-gluconat umfasst.

2. Mikronährstoffkombinationsprodukt nach Anspruch 1, **dadurch gekennzeichnet, dass** das Mikronährstoffkombinationsprodukt, bezogen auf 100 g des Mikronährstoffkombinationsproduktes, die Wirkstoffe umfasst:
a. 1-10 g, bevorzugt 5-9 g, Zink;
b. 100-300 mg, bevorzugt 150-200 mg, Kupfer.

3. Mikronährstoffkombinationsprodukt nach Anspruch 1 oder 2, **dadurch gekennzeichnet, dass** das Mikronährstoffkombinationsprodukt fest-, flüssig und/oder gelförmig vorliegt, vorzugsweise liegt das Mikronährstoffkombinationsprodukt in Präparatformen, ausgewählt aus der Gruppe umfassend Tabletten, Kapseln, Pulver, Granulat, Lösungen und/oder Brausetabletten vor.

4. Mikronährstoffkombinationsprodukt nach einem der vorherigen Ansprüche, **dadurch gekennzeichnet dass** das Mikronährstoffkombinationsprodukt Füllstoffe, Formentrennmittel und/oder Zerfallsbeschleuniger aufweist.

5. Mikronährstoffkombinationsprodukt nach einem der vorherigen Ansprüche, **dadurch gekennzeichnet, dass** das Mikronährstoffkombinationsprodukt Zinkgluconat, Kupfer(II)-gluconat, Cellulose, Lactose, Polyvinylpolypyrrolidon und/oder Magnesiumstearat aufweist.

6. Mikronährstoffkombinationsprodukt nach einem der vorherigen Ansprüche, **dadurch gekennzeichnet dass** das Mikronährstoffkombinationsprodukt, bezogen auf 100 g des Mikronährstoffkombinationsproduktes, die Wirkstoffe umfasst:
a. 7,1 g Zink;
b. 179 mg Kupfer.

7. Mikronährstoffkombinationsprodukt nach einem der vorherigen Ansprüche, **dadurch gekennzeichnet, dass** das Mikronährstoffkombinationsprodukt, bezogen auf eine Einzeldosis, die Wirkstoffe umfasst:
a. 10 mg Zink;
b. 250 µg Kupfer.

8. Verwendung eines Mikronährstoffkombinationsproduktes nach einem der vorherigen Ansprüche zur Herstellung eines Mittels zur diätetischen Verhütung und Behandlung von Augenkrankheiten, bevorzugt bei altersbedingter Makuladegeneration.

9. Verwendung eines Mikronährstoffkombinationsproduktes nach einem der vorherigen Ansprüche als Mittel zur Nahrungsergänzung.

10. Lebensmittel, insbesondere Nahrungsergänzungsmittel, umfassend Wirkstoffe gemäß einem der vorherigen Ansprüche.
